(19) **Europäisches Patentamt / European Patent Office / Office européen des brevets**

(11) **EP 4 711 472 A1**

(12) # EUROPEAN PATENT APPLICATION

(43) Date of publication:
**18.03.2026  Bulletin 2026/12**

(21) Application number: **25202574.7**

(22) Date of filing: **16.09.2025**

(51) International Patent Classification (IPC):
***C12Q 1/6827*** (2018.01)

(52) Cooperative Patent Classification (CPC):
(C-Sets available)
**C12Q 1/6827**　　　　　　　　　　　　　　(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **17.09.2024  IT 202400020632**

(71) Applicant: **Artemisia S.p.A.**
**00198 Roma (RM) (IT)**

(72) Inventors:
- **GIORLANDINO, Claudio**
  **ROMA (RM) (IT)**
- **MESORACA, Alvaro**
  **I-00198 ROMA (RM) (IT)**
- **CIMA, Antonella**
  **I-00198 ROMA (RM) (IT)**
- **MARGIOTTI, Katia**
  **I-00198 ROMA (RM) (IT)**
- **FABIANI, Marco**
  **I-00198 ROMA (RM) (IT)**

(74) Representative: **Pinnarò, Chiara et al**
**Praxi Intellectual Property S.p.A.**
**Via Leonida Bissolati, 20**
**00187 Roma (IT)**

(54) **DNA NANOBALL-BASED METHOD FOR THE DETECTION OF CHROMOSOMAL ANEUPLOIDIES AND COPY NUMBER VARIATIONS IN CIRCULATING FREE FETAL DNA**

(57)　The present invention relates to an improved method, in term of accuracy, efficiency, and reliability, for determining the probability of the risk of chromosomal and genetic disorders from free DNA of fetal origin. The invention utilizes Next Generation Sequencing (NGS) and bioinformatic analysis of obtained sequences to determine, in a sample of maternal plasma containing maternal and fetal DNA, the percentage of fetal DNA, alterations in chromosome numbers (fetal chromosomal aneuploidies), and variations in the number of copies of chromosomal regions (CNVs).

Pretreatment (30 min) → cfDNA Extraction (30 min) → Library preparation (120 min) → QC& Pooling (60 min) → Circularization (40 min) → Make DNB &Sequencing (~14h)

Figure 1

(52) Cooperative Patent Classification (CPC): (Cont.)

C-Sets
**C12Q 1/6827, C12Q 2531/125, C12Q 2535/122,
C12Q 2537/165, C12Q 2525/307**

**Description**

This innovation focuses on enhancing non-invasive prenatal testing (NIPT) methodologies through advanced Next Generation Sequencing (NGS) techniques and bioinformatics to accurately identify fetal chromosomal anomalies from maternal plasma.

**Field of the invention**

The invention relates generally to the field of diagnostics, specifically to the field of Non-Invasive Prenatal Diagnosis (NIPT). The invention utilizes Next Generation Sequencing (NGS) and bioinformatic analysis of obtained sequences to determine, in a sample of maternal plasma containing maternal and fetal DNA, the percentage of fetal DNA, alterations in chromosome numbers (fetal chromosomal aneuploidies), and variations in the number of copies of chromosomal regions (CNVs).

**Prior Art**

Cell-free DNA (cfDNA) extracted from peripheral blood of pregnant women is widely used for screening fetal chromosomal aneuploidies, including Down syndrome (trisomy 21, T21), Edwards syndrome (trisomy 18, T18), Patau syndrome (trisomy 13, T13), and sex chromosomal aneuploidies (SCAs). Lo et al., in 1997, first described fetal cfDNA in the plasma of pregnant women [1], and a prenatal testing method based on cfDNA was introduced in 2008 [2]. Compared to traditional serum screening, cfDNA testing has higher sensitivity, lower false positive rates, and higher positive predictive value (PPV) [3, 4]. Both the American College of Obstetricians and Gynecologists (ACOG) and the American College of Medical Genetics and Genomics (ACMG) have endorsed Non-Invasive Prenatal Testing (NIPT) as a routine screening option [5, 6]. The use of NIPT for detecting fetal aneuploidies has rapidly transformed the global landscape of prenatal screening, with the test being performed on millions of pregnant women worldwide [7, 8, 9, 10].

The performance and accuracy of NIPT, not only for common aneuploidies such as T21, T18, and T13 but also for other aneuploidies (autosomes and sex chromosomes), and CNVs, are crucial for improving the clinical utility of the test. Sensitivity and accuracy are very high for the three classical trisomies (T21, T18, and T13), but much less so for all other abnormalities in the number and structure of human chromosomes.

Principal objective of the present invention is, therefore, to provide an improved method suitable for rapid and early diagnosis of chromosomal abnormalities and CNVs of the fetus in a non-invasive manner, useful for providing more accurate, precise and safe indications without risks.

**Summary of the Invention**

It is object of the present invention to provide an improved method to determine, in a sample of maternal plasma containing maternal and fetal DNA, the percentage of fetal DNA, alterations in chromosome numbers (fetal chromosomal aneuploidies), and variations in the number of copies of chromosomal regions (CNVs). The method is based on the following steps:

**Sample Collection:** The method begins with obtaining a sample of peripheral blood and storing it in specially designed EDTA anticoagulation tubes, ensuring the preservation of circulating fetal DNA (cfDNA) integrity.

**cfDNA Extraction Protocol:** Through a series of optimized manual and/or automated steps, cfDNA is extracted with a high level of purity and yield, crucial for the subsequent steps of the testing process.

**Pioneering Library Preparation and Sequencing:** The process involves state-of-the-art library preparation techniques including either manual and/or automated adapter ligation and PCR amplification, preparing the cfDNA for highly accurate sequencing.

**Novel Circularization Process:** the process has a step of circularization of double-stranded DNA libraries. This method facilitates rolling circle amplification, a key differentiator that eliminates PCR-related errors and enhances the fidelity of sequencing.

**Advanced Analytical Techniques:** Following sequencing, a bespoke bioinformatics analysis is employed to interpret data with high precision, enabling the detection of chromosomal abnormalities with superior accuracy.

The methodology exhibits exceptional predictive values for detecting conditions like trisomies 21, 18, and 13, and sex chromosome aneuploidies (SCAs), boasting near-perfect sensitivity and specificity across extensive clinical trials. By providing more accurate results, the patented method minimizes the psychological and economic impacts of prenatal testing, offering expectant parents and clinicians a highly reliable screening tool. This patent represents a major leap forward in prenatal diagnostics, introducing a method that not only enhances the detection capabilities for fetal chromosomal abnormalities but also significantly reduces the risk and stress associated with traditional prenatal testing methods. This invention is poised to transform prenatal care by making early detection of fetal abnormalities more accessible and accurate.

**Brief description of the figures:**

    **Figure 1:** technical Workflow of the method according to the invention.
    **Figure 2:** Example of optimal cfDNA library result.
    **Figure 3:** DNA nanoball (DNB) formation and amplification process.

**Detailed Description of the Invention**

The invention provides improved in vitro processes for identifying the presence or absence of one or more fetal genetic variations, such as chromosomal abnormalities. These processes offer the advantages of being non-invasive, providing rapid results, and delivering results with high confidence, specificity, and sensitivity. The applications include non-invasive prenatal screening, copy number variation detection, and quality control tools for molecular biology methods related to cellular replication.

The method according to the present the invention has several aims as the determination of the presence or absence of a genetic variation and the prediction of fetal gender that can be determined based on sex chromosomes.

The methods can detect fetal chromosome abnormalities, which include the gain or loss of entire chromosomes or regions containing genes. Abnormalities include monosomies, trisomies, polysomies, deletions, duplications caused by unbalanced translocations, and microdeletions and microduplications also called copy number variations (CNVs).

CNVs are structural rearrangements of genomic sections caused by deletions, duplications, inversions, and translocations.

The method object of the present application is an in-vitro method, that is performed on blood obtained from a female at a gestational age appropriate for the specific test being conducted. The suitable gestational age for sample collection varies depending on the type of prenatal test performed. In certain embodiments, the pregnant female subject might be in the first trimester of pregnancy, which typically spans from conception to the end of the 12th week. During this period, many critical developmental processes occur, making it a key timeframe for early prenatal diagnostics.

In other embodiments, sample collection may take place during the second trimester of pregnancy, which extends from the beginning of the 13th week to the end of the 26th week. This period is characterized by continued fetal growth and development, and many prenatal tests can be effectively conducted during these weeks to monitor the health and development of the fetus.

For practical implementation, the invention allows to analyse samples obtained from a female at a gestational age 10, preferably from 10 to 13 weeks, and its able to analyse fetal DNA covering the entire period from early conception to full term.

**Library and sequencing Reads preparation**

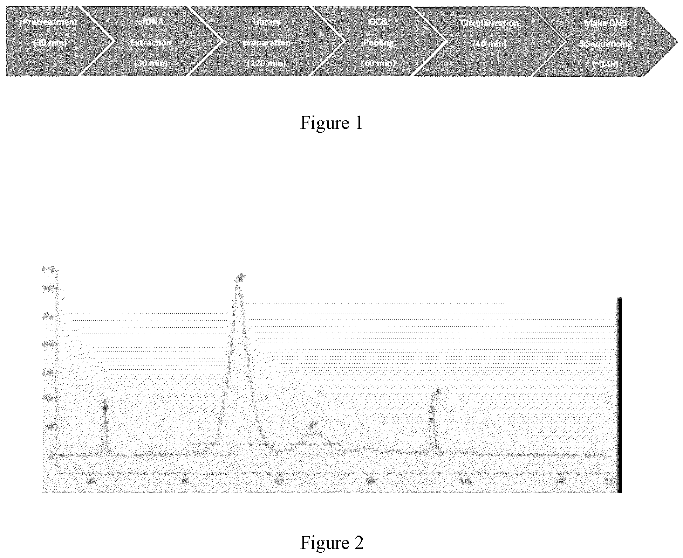

The invention relates to a method for analysing cell free fetal DNA from a peripheral blood sample, particularly from a pregnant woman, and system utilized for high-throughput sequencing methods that involve clonally amplified DNA templates (DNA Library) that are sequenced in a massively parallel fashion within a flow cell comprising the following steps depicted in Figure 1.

Such sequencing methods also can provide one of the most innovative aspects in terms of non-invasive prenatal screening, the use of circularized double-stranded DNA libraries.

Circularized double-stranded DNA libraries are specialized collections of DNA molecules that have been engineered to form continuous loops, where the ends of the DNA strands are covalently linked together. This circular structure contrasts with the more common linear DNA, where the ends are free. Said circularized double-stranded DNA is then processed to form DNA nanoballs.

Inventors found that Circularization of DNA offers several unique advantages that can be exploited in the method according to the invention and in particular in NIPT analysis, namely increased stability, resistance to exonuclease degradation since it lacks free ends that are susceptible to enzymatic attack. The process according to the invention implemented with circularized DNA unexpectedly allows for a superior performance and accuracy in detecting, not only for common aneuploidies such as T21, T18, and T13 but also for other aneuploidies (autosomes and sex chromosomes), microdeletions and microduplications, deletions, and CNVs.

Systems utilized for high-throughput sequencing methods are commercially available suitable for high-throughput sequencing of circular DNA Library, for example, Genetic Sequencer DNBSEQ platform.

**Samples:** Samples used are peripheral blood samples obtained from a female subject at a gestational age 1 to 45 weeks.

In a preferred embodiment 5 ml of peripheral blood obtained from a female at a gestational age 10 to 13 weeks are stored in an EDTA anticoagulation tube.

This specific tube choice stabilizes the blood and prevents the degradation of DNA in particular of circulating fetal DNA, which is essential for ensuring the accuracy of subsequent analyses.

**Plasma separation:**

Plasma separation can be done according to protocols know to the skilled in the art, preferably following the fundamental steps:

1. Precool the centrifuge to 4°C.
2. Transfer 1-1.5 ml whole blood to 1.5 ml tubes. Centrifuge the blood at 4500 rpm (2000 $\times$ g) for 10 min at 4°C. Transfer the supernatant to new tubes.
3. Centrifuge at 12000 rpm (16,000 $\times$ g) for 10 min at 4°C, then transfer supernatant to new tubes.

Plasma can be stored for up to 1 week at -25 ~ -18°C and up to 2 years at -70°C. and preferably should not undergo more than 2 freeze-thaw cycles.

Frozen samples should be thawed at room temperature and fully mixed before use.

**Cell free DNA Extraction:**

The extraction protocol is optimized to maximize cell free DNA yield and purity, in order to obtain a sample enriched in the fetal fraction DNA.

This step is critical to remove any potential contaminants that could interfere with the sequencing process. A nucleic acid may be extracted, isolated, purified by Automated System, and /or by Manual System (e.g., "by the hand of man") from its original environment (maternal blood and plasma). An isolated nucleic acid is provided with fewer non-nucleic acid components (e.g., protein, lipid) than the amount of components present in a source sample. Extraction and isolation of cfDNA can be obtained with automated and manual procedures. For example, automated systems are based on: Pre-packaged Reagents: The use of pre-packaged reagents reduces variability and enhances the reproducibility of the results. These reagents are prepared to ensure optimal conditions for the binding and isolation of cfDNA:

- Add 400 $\mu$l sample and 20 $\mu$l Proteinase K to Columns 1 and 7 of the 96-well plate. Pay attention to avoid cross-contamination.
- Place the 96 deep well plate into the nucleic acid extraction instrument. Load the magnetic bar sleeves, and ensure it fully envelops the magnetic bars.
- Set the program for automated extraction.

An automated nucleic acid extraction process is employed to handle the cfDNA, using magnetic bead technology for purification. This step is critical in maintaining high standards of purity and reducing manual handling errors.

Nucleic acids can be extracted using established manual methods, involving cell lysis through chemical, physical, or electrolytic means. Chemical methods typically use lysing agents followed by treatment with chaotropic salts. Physical methods include freeze/thaw cycles, grinding, or the use of cell presses. High salt lysis procedures, such as alkaline lysis, are also common.

All of these procedures are well known in the art.

**Library Preparation:**

The preparation of DNA libraries as well, as described in the follow protocol, can be carried out either manually or through automated machine, using identical procedures and reagents.

The manual method involves human operators performing each step of the protocol, while the automated method employs automated instruments to perform the same steps. To perform DNA libraries, initially is crucial to prepare DNA fragments for subsequent adapter ligation and amplification processes.

For this procedure, the Vazyme kit is referenced (Universal DNA Library Prep Kit for MGI). Afterwards adapter ligation is performed. The ligation of adapters is a critical step in DNA library preparation for sequencing. It involves attaching short, synthetic DNA sequences, known as adapters, to the ends of DNA fragments that have been prepared during the end preparation step. These adapters contain sequences necessary for binding the DNA to sequencing platforms and for the amplification of the DNA fragments. The ligation process is facilitated by enzymes such as DNA ligase, which form covalent bonds between the adapters and the DNA fragments, ensuring that they are securely attached for downstream applications. This step is essential for enabling the sequencing of the DNA fragments, as the adapters provide the necessary signals and binding sites for sequencing machinery. An adapted version of Vazyme kit (Universal DNA Library Prep Kit for MGI) was used. Purification of the reaction products was done by VAHTS DNA Clean Beads following manufactural procedure. The HiFi amplification of the library is a process designed to amplify the DNA fragments with high fidelity, ensuring accurate replication of the original sequences. This protocol involves the following steps:

**Preparation:**

Thaw the PCR Primer Mix

Equilibrate all components to room temperature before starting.

**Reaction Setup:**

In a sterile PCR tube, prepare the reaction solution as follows:

20 $\mu$l of purified adapter ligation products.
5 $\mu$l of PCR Primer Mix
25 $\mu$l of enzyme.

**PCR Amplification:**

Place the PCR tube in a thermal cycler and perform the following cycling conditions:

Initial denaturation at 95°C for 3 minutes.
Denaturation at 98°C for 20 seconds.
Annealing at 60°C for 15 seconds.
Extension at 72°C for 30 seconds.
Repeat steps 2 to 4 for 12 cycles.
Final extension at 72°C for 5 minutes.
For library quality control, Agilent 2100 Bioanalyzer, was used (Figure 2).

**Circularization:**

The circularization of single-stranded DNA is a crucial step in the preparation of DNA for rolling circle replication. During this process, linear DNA fragments are converted into circular molecules. This is important because the circular DNA templates are required for the rolling circle replication process. The circularization ensures that the DNA fragments can be continuously replicated, leading to the production of a large number of copies from a single template. This step helps to stabilize the DNA and prepares it for the next stage of amplification. Nucleic acid circularization can be done with different methods we show herein an example of an adapted protocol extracted from VAHTS Circularization KIT manual.

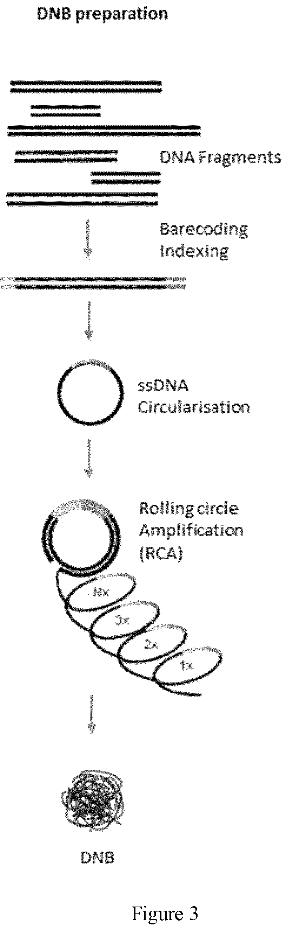

Once the DNA is circularized, it undergoes rolling circle replication (RCR) to form DNA nanoballs (DNBs) (Figure 3).

DNBs are highly compact, multi-copy arrays of the original DNA sequence. The formation of DNBs is significant for several reasons: High Yield and Uniformity: The RCR process amplifies the circular DNA into a large number of copies without the use of PCR, which helps to avoid the accumulation of errors and biases that can occur during PCR amplification. Efficiency in Sequencing: DNBs provide a high-density, high-fidelity template for sequencing platforms. Their uniform size and structure facilitate efficient loading onto sequencing chips, enhancing the throughput and accuracy of sequencing. Error Minimization: The PCR-free nature of RCR reduces the introduction of amplification errors, ensuring that the sequences remain true to the original template.

**The Circularization and DNA Nanoballs protocol involves the following steps:**

Denaturation, and Formation of DNA Nanoballs (DNBs) are prepared by the following steps: 20 $\mu$l of reaction product from the previous step and 20 $\mu$l of DNB Buffer (VAHTS Circularization KIT). Mix gently by vertexing, centrifuge for 5 seconds, and place the mix into a PCR machine for primer hybridization. PCR machine settings: Hot lid at 105°C, followed by 95°C, 65°C, 40°C, and 4°C. DNB Reaction and quantification were done an adapted by following the manufactural indication (VAHTS Circularization KIT).

**Sequencing**

The sequencing step is preferably performed using Next Generation Sequencing NGS techniques. Different sequencers and sequencing technique can be used according to the invention.

In a preferred embodiment Genetic Sequencer DNBSEQ platform is used.

In another preferred embodiment the MGI400 sequencer is used.

As an example of sequencing the following protocol is used:

Sequencing Preparation: Quantify 2 $\mu$l of DNB using the Qubit® ssDNA Assay Kit and Qubit® Fluorometer. Ensure a minimum DNB concentration of 8 ng/$\mu$l for sequencing. Load the pools automatically into the MGI400 sequencer for sequencing. Overall, the circularization and formation of DNBs are essential steps in preparing high-quality DNA libraries for next-generation sequencing, and NIPT analysis contributing to the accuracy, efficiency, and reliability of the prenatal test procedure.

**Analysis Pipeline**

The sequencing process begins with the extraction of cell-free DNA from maternal blood, deriving both maternal and fetal DNA, with the fetal fraction originating from the placenta.

Sequencing generates paired-end 100 base pair reads stored in FASTQ format, a text-based format that stores nucleotide sequences and corresponding quality scores. Each sequence is accompanied by a Phred quality score, providing a logarithmic scale of the probability of an incorrect base call, influencing subsequent alignments against reference genomes. Following sequencing, an initial quality control check is conducted to ensure read integrity and accuracy. This step identifies low-quality reads, adapter contamination, and PCR duplicates, which can introduce biases and reduce downstream analysis reliability. Detailed summaries and visualizations of read quality metrics enable the identification and mitigation of these issues.

The pipeline progresses to the alignment of reads using Bowtie2, employing the Burrows-Wheeler Transform (BWT) algorithm for efficient mapping of reads to the genome. This reduces computational resources required for alignment, enabling rapid and accurate mapping of millions of short reads. The output from this alignment step is stored in the Sequence Alignment Map (SAM) format, including a header section with metadata about the alignments and an alignment section containing detailed information for each read, including its mapping position, quality, and alignment information encoded in the CIGAR string (Concise Idiosyncratic Gapped Alignment Report).

Refinement of the aligned data involves removing reads that map to multiple locations in the genome, known as multiple mapping reads, to ensure specificity to a single genomic location, enhancing reliability. Identification and removal of duplicate reads, often resulting from PCR amplification artifacts, prevent artificially inflated read counts and biases in quantitative analyses.

Aligned, filtered, and deduplicated reads are then converted into the Binary Alignment Map (BAM) format. BAM files are a compressed binary version of the Sequence Alignment Map (SAM) format, designed to efficiently store large amounts of sequence alignment data. The SAM format is a tab-delimited text format that consists of a header section and an alignment section. The header section contains information about the reference genome, including the reference sequences, their lengths, and any optional metadata. The alignment section contains alignment information for each read, such as the read name, the sequence, the quality scores, the reference sequence name, the alignment position, the mapping quality, and the CIGAR string. The CIGAR string encodes the alignment of the read to the reference sequence, indicating matches, insertions, deletions, and other features.

Converting reads into BAM format significantly reduces file size through binary compression, making it more efficient for storage and transmission. The binary nature of BAM files also allows for efficient random access to the data, which is crucial for downstream analysis tasks that require quick retrieval of specific alignment information. BAM files are accompanied by an index file (BAI), which provides an index of the BAM file, allowing for rapid access to alignment data for specific genomic regions without having to scan through the entire file. The BAI file enables tools to quickly locate and retrieve alignments for a particular region, streamlining processes such as variant calling, visualization, and other genomic analyses.

**Normalization and Data Adjustment**

Normalization is crucial for adjusting technical variability, such as differences in sequencing depth and biases introduced by GC content. Normalization involves calculating the Normalized Chromosome Value (NCV), which is the ratio of reads aligning to a particular chromosome to reads aligning to a reference chromosome or set of chromosomes.

Adjustments account for GC content variations, ensuring accurate quantification across different genomic regions.

To correct for GC content bias, the pipeline first calculates the GC content of each sequencing read, determining the percentage of guanine (G) and cytosine (C) bases in each read. The GC content distribution of the entire dataset is then analyzed to identify any systematic biases. The pipeline adjusts read counts based on GC content by binning reads according to their GC content and comparing the read counts in each bin to expected counts based on a reference genome. If certain GC content bins have higher or lower read counts than expected, a correction factor is applied to normalize these counts. This factor is the ratio of the expected read count to the observed read count for each GC content bin, ensuring that regions with atypical GC content do not disproportionately influence the overall read count.

Additionally, the pipeline corrects for potential biases introduced during read alignment. Sequencing reads can sometimes align more efficiently to certain regions of the genome due to sequence complexity or the presence of repetitive elements. The pipeline uses a reference dataset to model alignment efficiency across different genomic regions. By comparing the observed alignment rates to these models, the pipeline adjusts read counts to account for regions with higher or lower alignment efficiency.

Furthermore, the pipeline addresses potential PCR amplification biases. PCR can preferentially amplify certain DNA fragments over others, leading to skewed read counts. The pipeline models the expected distribution of read counts based on known amplification biases and applies correction factors to the observed read counts. These factors are derived from control experiments that measure the extent of PCR bias for different sequences.

Masking repetitive and low-complexity regions ensures that only unique, informative reads are considered in the analysis, reducing noise and improving the accuracy of fetal fraction estimates and the detection of aneuploidies. By integrating these comprehensive corrections, the pipeline ensures that the final read counts used for fetal fraction estimation and aneuploidy detection are as accurate and unbiased as possible. This detailed approach enhances the reliability of the results, providing a robust foundation for subsequent analyses.

**Chromosome Dosage Assessment**

Z-score calculations derived from normalized read counts are crucial for assessing the presence of aneuploidies in non-invasive prenatal testing (NIPT). The z-score determines how far a chromosome's read count deviates from the expected baseline, indicating potential chromosomal abnormalities. This statistical measure is essential for identifying deviations in chromosome dosage, such as trisomies or monosomies, which can lead to genetic disorders. In the context of NIPT, the raw read counts for each chromosome are first normalized to account for technical variability, including sequencing depth and GC content biases. This normalization ensures that read counts are comparable across different samples and regions. Subsequently, the mean and standard deviation of the read counts for each chromosome are calculated from a reference dataset representing the expected baseline for euploid conditions, derived from a large number of control samples.

For each chromosome in the test sample, the observed read count is compared to the reference mean and standard deviation using the z-score formula. This calculation determines the extent to which the read count deviates from the expected value in terms of standard deviations.

A z-score exceeding +3 indicates a significant deviation from the mean, suggesting the presence of an extra copy of the chromosome (trisomy).

Conversely, a z-score significantly lower than the mean (e.g., less than -3) suggests a missing copy of the chromosome (monosomy). These thresholds are statistically significant, with a z-score of +3 corresponding to a deviation that is highly unlikely to occur by chance.

The z-score (Z) is calculated using the formula:

$$Z = \frac{X - \mu}{\sigma}$$

15

where:

- X is the observed read count for a particular chromosome,
- $\mu$ is the mean read count for that chromosome based on a proprietary baseline,
- $\sigma$ is the standard deviation of the read counts for that chromosome.

This z-score calculation is highly sensitive to variations in read counts, enabling the detection of even subtle deviations from the expected chromosome dosage.

**Fetal Sex Determination**

The pipeline determines fetal sex through advanced analysis of sequencing data, leveraging Y chromosome read counts and genomic features. The method relies on a proprietary machine learning classifier trained to estimate the probability of a male fetus based on these features. The process begins by mapping sequencing reads to the reference human genome, with a specific focus on the Y chromosome. The presence of Y chromosome reads is a direct indicator of a male fetus, while their absence suggests a female fetus. In addition to read counts, the GC content of the Y chromosome is used to account for potential sequencing biases, particularly those related to GC content that can affect read mapping and coverage.

Key features are extracted from the genomic data, including the count of reads aligned to the Y chromosome (chrY_count) and the GC content of the Y chromosome (chrY_gc). These features are essential inputs for the machine learning classifier based on logistic regression, which has been pre-trained on data from known male and female fetal samples. The classifier uses these inputs to predict fetal sex, employing a model that considers the relationship between Y chromosome presence and sequencing efficiency affected by GC content.

Once the necessary features are extracted, the pipeline normalizes and formats them for input into the classifier. The machine learning model generates two key outputs: a binary prediction indicating the fetal sex (male or female) and a probability score that quantifies the likelihood of the fetus being male. This probability score provides an additional layer of confidence for the prediction, allowing healthcare professionals to assess the certainty of the result.

The pipeline outputs these results in a tabular report, including both the predicted fetal sex and the associated probability of a male fetus. This information can then be used in clinical decision-making, enhancing the reliability of prenatal diagnostics. By incorporating machine learning and GC content correction, the pipeline reduces sequencing biases and ensures accurate sex determination, even under varying sequencing conditions and fetal fractions.

**Fetal Fraction Estimation**

The fetal fraction, which is the proportion of cell-free DNA (cfDNA) in maternal blood originating from the fetus, is estimated using the PREFACE method (PREdict FetAl ComponEnt). It is a sophisticated computational pipeline designed to accurately estimate fetal fraction from shallow-depth whole-genome sequencing data (sWGS) without requiring additional experimental assays, making it ideal for routine testing. The method begins with an unsupervised learning step, where principal component analysis (PCA) is applied to autosomal regions, excluding aneuploidy-prone regions such as chromosomes 13, 18, and 21. This step captures variance in the sequencing data, which includes contributions from fetal DNA, and is applicable to both male and female fetuses. In the subsequent supervised learning phase, the model is trained on a large cohort of male samples, where fetal fraction (FFY) is determined based on the read depth of the Y chromosome. In-house sequencing data is used in order to reduce variability and adapt to specific experimental conditions. A neural network uses this training to weigh the principal components, which capture fetal-induced variance in the autosomal data. Once trained, the model is applied to both male and female samples. For female fetuses, the model predicts the fetal fraction using the same learned associations between principal components and fetal DNA without relying on Y-chromosome reads or sex-specific markers. The method uses bin-wise log2 ratios of observed versus expected read counts to account for noise and variability in the sWGS data. By utilizing autosomal data in combination with machine learning techniques, the method provides fetal fraction predictions for both male and female samples within the NIPT pipeline.

**Detection of Microdeletions and Microduplications**

Detecting microdeletions and microduplications, deletions and smaller-scale chromosomal abnormalities, involves a comprehensive analysis of specific genomic regions or 'tiles' to identify variations in DNA copy number relative to the reference genome. This detection process uses several methodologies to ensure accurate identification and characterization of these structural variations.

The first method employed is read depth analysis. This technique involves counting the number of sequencing reads that map to specific genomic regions. By comparing the observed **read depth** to a reference dataset, the pipeline infers copy number variations. Significant increases in read depth suggest duplications, while decreases indicate deletions. This comparison provides an initial assessment of potential copy number variations across the genome.

**Paired-end mapping** is another crucial method. This technique analyzes the distances and orientations of paired-end reads, which are sequences generated from both ends of a DNA fragment. By examining the expected distances and orientations of these paired-end reads based on the reference genome, deviations from these expectations indicate potential structural variations such as duplications or deletions. This method allows for precise localization of breakpoints, enhancing the resolution of structural variant detection.

**Split-read analysis** further refines the detection of structural variations by identifying reads that span the breakpoints of these variations. This approach involves aligning portions of a read to different genomic locations, thereby pinpointing the exact sites of deletions or duplications. Split-read analysis is particularly effective for detecting smaller and more complex structural variations that might be missed by read depth or paired-end mapping alone. **Copy number variation (CNV) analysis** involves comparing the copy numbers of DNA segments between the sample and the reference genome. This method uses statistical models and computational algorithms to identify regions with significant deviations in copy number, indicating the presence of microdeletions or microduplications. Specifically, the CNV analysis process employs a combination of Hidden Markov Models (HMM) and segmentation algorithms. The HMM is used to model the underlying copy number states and to detect transitions between different states, such as normal copy number, deletion, and duplication. Segmentation algorithms then partition the genome into contiguous segments with uniform copy number, allowing for precise identification of regions with abnormal copy numbers. By integrating these methodologies - read depth analysis, paired-end mapping, split-read analysis, and CNV analysis using HMM and segmentation algorithms - the detection process ensures a comprehensive approach to identifying microdeletions and microduplications. This integration provides high sensitivity and specificity in detecting these smaller-scale chromosomal abnormalities. Normalized read counts and z-scores are calculated to quantify significant deviations from the established baseline, facilitating the accurate detection of genomic alterations that are crucial for diagnosing various genetic conditions not detectable by traditional chromosome-wide aneuploidy screening.

## Reporting

The result of this comprehensive pipeline is a detailed report that provides healthcare providers with information about the fetal health status, enabling informed decision-making in prenatal care. This report not only includes findings on potential chromosomal abnormalities, fetal sex, and microdeletions but also contextualizes these findings within the landscape of the existing medical literature, providing a robust foundation for prenatal diagnostic decisions.

## EXAMPLES

**Performance of circularized fetal DNA in detecting trisomies 21, 18, and 13**

Evaluation of the performance of our fetal DNA test in detecting trisomies 21, 18, and 13 and SCAs in a cohort of 7,020 singleton pregnancies, based on the confirmatory follow-up, shows a PPV of 99.8% for trisomy 21, 96.73% for trisomy 18, and 95.22% for trisomy 13 (Table 1). Follow-up results for SCAs estimated PPV of 97.14 %, (Table 1). Other NIPT results performed and obtained in data available for 36,000 single pregnancies (11) based on technologies different than the one described in the invention (VeriSeq NIPT Solution, Illumina) shows a decreased magnitude in terms of PPV in comparison with ours results (Table 2). PPV is calculated on the confirmatory follow-up and represent the probability that fetuses with a positive test truly have the genetic disorder. Positive predictive values are important and critical parameters as its accuracy and high values directly correlate with reduced costs and mitigated psychological challenges experienced by prospective mothers. Given that a false positive test can prompt the requirement for clinical and diagnostic investigations, resulting in significant economic losses and psychological distress, it is crucial to acknowledge that even slight discrepancies between different technologies (as presented in Table 1 and Table 2) can profoundly influence clinical practices. The sequencing and analysis method according to the invention offers a more rapid turnaround times and based on the discussed results higher and more accurate screening test (Table1 and Table 2).

### Table 1. SINGLE PREGNANCIES NIPT TEST AFTER DNA CIRCULARIZATION

| | Trisomy 21 | Trisomy 18 | Trisomy 13 | SCA |
|---|---|---|---|---|
| | | | | |
| **N° FETALDNA 7,020** | | | | |
| **Sensitivity** | 100% | 100% | 100% | 100% |
| **Specificity** | 99.99% | 99.99% | 99.99% | 99.97% |
| **PPV** | **99.8%** | **96.73%** | **95.22%** | **97.14%** |
| LEGEND: PPV: POSITIVE PREDICTIVE VALUE SCA: SEX CHROMOSOME ANEUPLOIDIES | | | | |

**Table 2. SINGLE PREGNANCIES NIPT TEST COMPETITOR (VeriSeq NIPT Solution, Illumina)**

| n = 36,000 | TP | FP | TN | Sensitivity | Specificity | PPV |
|---|---|---|---|---|---|---|
|  |  |  |  |  |  |  |
| T21 | 247 | 2 | 35,410 | 100 | 99.99 | 99.20 |
|  |  |  |  | 247/247 | 35,410/35,412 | 247/249 |
|  |  |  |  |  |  |  |
| T18 | 62 | 6 | 35,591 | 100 | 99.98 | 91.20 |
|  |  |  |  | 62/62 | 35,591/35,597 | 62/68 |
|  |  |  |  |  |  |  |
| T13 | 27 | 5 | 35,627 | 100 | 99.99 | 84.40 |
|  |  |  |  | 27/27 | 35,627/35,632 | 27/32 |
|  |  |  |  |  |  |  |
| SCA | 117 | 18 | 35,524 | 100 | 99.95 | 86.7 |
|  |  |  |  | 117/117 | 35,524/35,542 | 117/135 |
| LEGEND: TP true positive, FP false positive, FN false negative | | | | | | |

**Bibliography**

1) YM, Corbetta N, Chamberlain PF et al (1997) Presence of fetal DNA in maternal plasma and serum. Lancet 350(9076):485-487

2) Chiu RW, Chan KC, Gao Y et al (2008) Noninvasive prenatal diagnosis of fetal chromosomal aneuploidy by massively parallel genomic sequencing of DNA in maternal plasma. Proc Natl Acad Sci U S A 105(51):20458-20463

3) Bianchi DW, Rava RP, Sehnert AJ (2014) DNA sequencing versus standard prenatal aneuploidy screening. N Engl J Med 371(6):578

4) Norton ME, Jacobsson B, Swamy GK et al (2015) Cell-free DNA analysis for noninvasive examination of trisomy. N Engl J Med 372(17):1589-1597

5) Gregg AR, Skotko BG, Benkendorf JL et al (2016) Noninvasive prenatal screening for fetal aneuploidy, 2016 update: a position statement of the American College of Medical Genetics and Genomics. Genet Med 18(10):1056-1065

6) Committee Opinion No (2015) 640: Cell-Free DNA Screening For Fetal Aneuploidy. ObstetGynecol 126(3):e31-e37

7) van der Meij KRM, Sistermans EA, Macville MVE et al (2019) TRIDENT-2: National implementation of genome-wide non-invasive prenatal testing as a first-tier screening test in the Netherlands. Am J Hum Genet 105(6): 1091-1101

8) Guy C, Haji-Sheikhi F, Rowland CM et al (2019) Prenatal cell-free DNA screening for fetal aneuploidy in pregnant women at average or high risk: Results from a large US clinical laboratory. Mol Genet Genomic Med 7(3):e545

9) La Verde M, De Falco L, Torella A et al (2021) Performance of cell-free DNA sequencing-based non-invasive prenatal testing: experience on 36,456 singleton and multiple pregnancies. BMC Med Genomics 14(1):93

10) Soster E, Boomer T, Hicks S et al (2021) Three years of clinical experience with a genome-wide cfDNA screening test for aneuploidies and copy-number variants. Genet Med 23(7):1349-1355

11) Marco La Verde et al. Performance of cell-free DNA sequencing-based non-invasive prenatal testing: experience on 36,456 singleton and multiple pregnancies BMC Med Genomics. 2021 Mar 30;14(1):93. doi: 10.1186/s12920-021-00941-y.

**Claims**

1. A method for determining the probability of the risk of chromosomal and genetic disorders from free DNA of fetal origin comprising the following step:

   - storing a blood sample obtained from a female of gestational age comprised between 10 and 13 weeks in EDTA anticoagulation tube,
   - performing a plasma separation step,
   - extracting the cell free DNA from the plasma obtained,
   - preparing DNA fragments and ligating said fragments with adapters sequences,
   - performing a circularization and rolling circle replication and amplification of said fragments to form DNA nanoballs (DNBs),
   - processing said nanoballs through a sequencer,
   - obtaining the sequencing data in FASTQ format comprising the biological sequences and the quality scores of said biological sequences;
   - converting said sequencing from said FASTQ format to the SAM format, in which said biological sequences are aligned with a reference genome;
   - converting said sequencing from the SAM format to the BAM format, in which the data contained in said SAM format are compressed by conversion into binary code;
   - counting and categorizing the READ sequenced from said SAM format, in which each READ is categorized as mapped on each chromosome or mapped on all autosomes;
   - calculating the mean and standard deviation of the read counts for each chromosome from a reference dataset representing the baseline for euploid conditions;
   - comparing the read count for each chromosome in the test sample to the reference mean and standard deviation and calculating the z score with the formula

$$Z = \frac{X - \mu}{\sigma}$$

   where:

   - X is the observed read count for a particular chromosome,
   - $\mu$ is the mean read count for that chromosome based on a reference baseline,
   - $\sigma$ is the standard deviation of the read counts for that chromosome.

   wherein a z score > 3 indicates a trisomy and a z-score significantly lower than the mean indicates a monosomy.

2. Method according to claim 1, wherein the sample is obtained from a female of gestational age comprised between 10 and 13 weeks.

3. Method according to claim 1 or 2, wherein said biological sequences are aligned with a reference genome, using the Burrows-Wheeler algorithm.

4. Method according to any one of the preceding claims, wherein the step of sequencing is carried out by means of Next Generation Sequencing NGS techniques.

5. Method according to any one of the preceding claims further comprising the step of calculating the fetal DNA fraction in the sample using the read depth of the Y chromosome and applying a model trained to assess the fetal fraction in samples using bin-wise log2 ratios of observed vs. expected read counts of said Y chromosome to account for noise and variability in whole-genome sequencing data.

6. Method according to any one of the preceding claims further comprising the step of detecting Microdeletions and Microduplications by using read depth analysis in combination with paired-end mapping, split-read analysis and copy number variation analysis, wherein the copy number variation analysis employs a combination of Hidden Markov Models (HMM) and segmentation algorithms.

Figure 1

Figure 2

## DNB preparation

DNA Fragments

Barecoding
Indexing

ssDNA
Circularisation

Rolling circle
Amplification
(RCA)

Nx

3x

2x

1x

DNB

Figure 3

Europäisches
Patentamt

European
Patent Office

Office européen
des brevets

**EUROPEAN SEARCH REPORT**

**Application Number**

**EP 25 20 2574**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2019/224668 A1 (ARTEMISIA S P A [IT]) 28 November 2019 (2019-11-28) * page 12, line 5 - line 7; claim 1 * * page 4, line 10 - page 5, line 7 * ----- | 1-6 | INV. C12Q1/6827 |
| Y | XU CHENMING ET AL: "Genetic deconvolution of fetal and maternal cell-free DNA in maternal plasma enables next-generation non-invasive prenatal screening", CELL DISCOVERY, vol. 8, no. 1, 13 December 2022 (2022-12-13), XP093202731, GB ISSN: 2056-5968, DOI: 10.1038/s41421-022-00457-4 * page 17, right-hand column, paragraph 1 * * page 19, right-hand column, paragraph 1 * ----- | 1-6 | |
| A | S. C. Y. YU ET AL: "Size-based molecular diagnostics using plasma DNA for noninvasive prenatal testing", PROCEEDINGS OF THE NATIONAL ACADEMY OF SCIENCES, vol. 111, no. 23, 19 May 2014 (2014-05-19), pages 8583-8588, XP055209819, ISSN: 0027-8424, DOI: 10.1073/pnas.1406103111 * page 8588, right-hand column, paragraph 4 * ----- -/-- | 1-6 | **TECHNICAL FIELDS SEARCHED (IPC)** C12Q |

The present search report has been drawn up for all claims

| Place of search | Date of completion of the search | Examiner |
|---|---|---|
| The Hague | 15 January 2026 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

EPO FORM 1503 03.82 (P04C01)

Europäisches Patentamt
European Patent Office
Office européen des brevets

# EUROPEAN SEARCH REPORT

**Application Number**

EP 25 20 2574

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | R. W. K. CHIU ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES (PNAS), vol. 105, no. 51, 23 December 2008 (2008-12-23), pages 20458-20463, XP055284693, ISSN: 0027-8424, DOI: 10.1073/pnas.0810641105 * the whole document * -& R. W. K. CHIU ET AL: "Supplementary Information for XP002620454 (Title: Noninvasive prenatal diagnosis of fetal chromosomal aneuploidy by massively parallel genomic sequencing of DNA in maternal plasma)", PROCEEDINGS OF THE NATIONAL ACADEMY OF SCIENCES (PNAS), vol. 105, no. 51, 10 December 2008 (2008-12-10), pages 20458-20463, XP055024153, ISSN: 0027-8424, DOI: 10.1073/pnas.0810641105 * page 1, right-hand column, paragraph 3 * ----- | 1-6 | |
| A | CN 118 006 738 A (GUANGZHOU VISION GENE TECH CO LTD ET AL.) 10 May 2024 (2024-05-10) * the whole document * ----- | 1-6 | **TECHNICAL FIELDS SEARCHED (IPC)** |

The present search report has been drawn up for all claims

| Place of search | Date of completion of the search | Examiner |
|---|---|---|
| The Hague | 15 January 2026 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 25 20 2574

This annex lists the patent family members relating to the patent documents cited in the above-mentioned European search report.
The members are as contained in the European Patent Office EDP file on
The European Patent Office is in no way liable for these particulars which are merely given for the purpose of information.

15-01-2026

| Patent document cited in search report | | Publication date | Patent family member(s) | | Publication date |
|---|---|---|---|---|---|
| WO 2019224668 A1 | | 28-11-2019 | EP | 3797418 A1 | 31-03-2021 |
| | | | ES | 2989171 T3 | 25-11-2024 |
| | | | WO | 2019224668 A1 | 28-11-2019 |
| CN 118006738 A | | 10-05-2024 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YM, CORBETTA N ; CHAMBERLAIN PF et al.** Presence of fetal DNA in maternal plasma and serum.. *Lancet*, 1997, vol. 350 (9076), 485-487
- **CHIU RW ; CHAN KC ; GAO Y et al.** Noninvasive prenatal diagnosis of fetal chromosomal aneuploidy by massively parallel genomic sequencing of DNA in maternal plasma.. *Proc Natl Acad Sci U S A*, 2008, vol. 105 (51), 20458-20463
- **BIANCHI DW ; RAVA RP ; SEHNERT AJ.** DNA sequencing versus standard prenatal aneuploidy screening.. *N Engl J Med*, 2014, vol. 371 (6), 578
- **NORTON ME ; JACOBSSON B ; SWAMY GK et al.** Cell-free DNA analysis for noninvasive examination of trisomy. *N Engl J Med*, 2015, vol. 372 (17), 1589-1597
- **GREGG AR ; SKOTKO BG ; BENKENDORF JL et al.** Noninvasive prenatal screening for fetal aneuploidy, 2016 update: a position statement of the American College of Medical Genetics and Genomics.. *Genet Med*, 2016, vol. 18 (10), 1056-1065
- Cell-Free DNA Screening For Fetal Aneuploidy. ObstetGynecol. *Committee Opinion No (2015) 640*, vol. 126 (3), e31-e37
- **VAN DER MEIJ KRM ; SISTERMANS EA ; MACVILLE MVE et al.** TRIDENT-2: National implementation of genome-wide non-invasive prenatal testing as a first-tier screening test in the Netherlands.. *Am J Hum Genet*, 2019, vol. 105 (6), 1091-1101
- **GUY C ; HAJI-SHEIKHI F ; ROWLAND CM et al.** Prenatal cell-free DNA screening for fetal aneuploidy in pregnant women at average or high risk: Results from a large US clinical laboratory.. *Mol Genet Genomic Med*, 2019, vol. 7 (3), e545
- **LA VERDE M ; DE FALCO L ; TORELLA A et al.** Performance of cell-free DNA sequencing-based non-invasive prenatal testing: experience on 36,456 singleton and multiple pregnancies.. *BMC Med Genomics*, 2021, vol. 14 (1), 93
- **SOSTER E ; BOOMER T ; HICKS S et al.** Three years of clinical experience with a genome-wide cfDNA screening test for aneuploidies and copy-number variants.. *Genet Med*, 2021, vol. 23 (7), 1349-1355
- **MARCO LA VERDE et al.** Performance of cell-free DNA sequencing-based non-invasive prenatal testing: experience on 36,456 singleton and multiple pregnancies. *BMC Med Genomics.*, 30 March 2021, vol. 14 (1), 93